# EUROPEAN PATENT SPECIFICATION

(11) **EP 4 225 170 B1**
(45) Date of publication and mention of the grant of the patent: **12.08.2026**
(21) Application number: 21794240.8
(22) Date of filing: 04.10.2021
(51) Int. Cl.: A61B 17/34, A61F 9/007

(54) **A TROCAR MODULE, A FLUID CONNECTOR AND METHODS**
TROKARMODUL, FLÜSSIGKEITSVERBINDER UND VERFAHREN
MODULE DE TROCART, RACCORD DE FLUIDE ET PROCÉDÉS

(30) Priority: 05.10.2020 NL 2026624
(43) Date of publication of application: 16.08.2023
(73) Proprietor: D.O.R.C. Dutch Ophthalmic Research Center (International) B.V., 3214 VN Zuidland (NL)
(72) Inventor: MACKAY, Alan William, 3214 VN Zuidland (NL); DE LANGE, Ronald, 3214 VN Zuidland (NL)
(74) Representative: V.O.
(86) International application number: PCT/NL2021/050601
(87) International publication number: WO 2022/075840

(56) References cited:
- WO-A1-2016/202332
- WO-A1-2020/047330
- WO-A2-2014/059420
- DE-B4- 102009 032 188
- US-A1- 2008 033 462
- US-A1- 2009 234 292
- US-B2- 8 343 106

## Description

The invention relates to a trocar module for ophthalmic surgery, comprising a tubular shaped cannula having a distal part for insertion through the eye's sclera and a proximal part for receiving a capillary tube of a fluid connector in a coupled state with said fluid connector, and a valve unit arranged at the proximal part of the cannula for sealingly surrounding the capillary tube in the coupled state.

Trocar modules are generally known in the field of ophthalmic surgery for providing access to the interior of the eye, via an insert opening, either for irrigation, fluid exchange or for surgical tools to illuminate, cut, remove or otherwise manipulate tissue. The tubular shaped cannula traverses the sclera of the eye, while the valve unit sealingly surrounds a capillary tube of a fluid connector coupled to the trocar module, in case of fluid exchange such as an irrigation line or an aspiration line for flushing the eye.

At a proximal side, the cannula is typically tapered for aligning the capillary tube of the fluid connector into the cannula. In a process of coupling the fluid connector with the cannula, the capillary tube is inserted, via the tapered portion, into the cannula beyond said tapered portion until the capillary tube extends sufficiently enough into the distal part of the cannula. Then, the capillary tube also traverses the valve unit that sealingly surrounds said capillary tube.

In recent developments, designs of ophthalmic surgery instruments miniaturize decreasing their effective diameter at structures where they pass the eye's sclera, increasing their gauge. However, when decreasing the capillary tube's internal diameter, also its operational fluid flow reduces which might be disadvantageous in terms of surgical performance. Higher irrigation pressure values may be needed with a reduced chamber stability.

It is therefore a need to provide a trocar module that may be implemented with small dimensions while facilitating a relatively large flow rate.

It is an object of the present invention to provide a trocar module for ophthalmic surgery wherein the exterior diameter is relatively small while maintaining a relatively large flow rate. Thereto, according to the invention, a trocar module for ophthalmic surgery according to claim 1 is provided.

By applying a tubular shaped cannula having a double tapered internal profile having an intermediate tube portion therebetween, the distal end of the capillary tube can be shifted, via the first tapered section towards the second tapered section. From the location where the capillary tube is located the second tapered section tapers towards the distal end of the cannula having the smallest internal diameter. Then, the capillary tube can safely be coupled to the cannula while the interior diameter of the capillary tube can still be substantially the same or greater than the inner diameter of the cannula distal part, resulting in a flow structure wherein the capillary tube does not extend or protrude into the smallest diameter structure of the cannula, its distal part. Then, the flow rate, generally limited by the passage having smallest internal diameter of the whole flow path, can be relatively large allowing a relatively low fluid pressure and a stable intra ocular pressure IOP, while, on the other hand, the exterior of the cannula can be relatively small to minimize wound trauma. The flow passage in the distal part of the cannula having a single wall, contrary to the double wall that is present in the known trocar module coupled to fluid connector, viz. the wall of the capillary tube and the wall of the cannula distal part.

By applying the double tapered structure, respective elements engage each other opening up the flow path, thereby resulting in a design having significantly improve flow performance for the same back-pressure. Then, a high-flow capability is obtained without a requirement to remove the valve unit. The double tapered structure, in particular, the intermediate tube section between both tapered sections, results in a design providing a reliable coupling force.

It is noted that patent publication US 2009/234292 A1 discloses a seal for use in a trocar, particularly for a trocar for eye surgery, having a seal element, with a seal opening, and an instrument opening. It is further noted that patent publication WO 2014/059420 A2 discloses an irrigating cannula including a hub having an inner passage and a side port extending through the hub and connecting to the inner passage.

Advantageously, an inner diameter of the intermediate tube portion is substantially the same or greater than an outer diameter of the cannula distal part, such that the inner diameter of the capillary tube of the fluid connector can be equal or larger than the inner diameter of the cannula distal part.

Preferably, the distal part of the tubular shaped cannula has an exterior surface that is at least partly provided with laser fabricated texture structures, thereby improving retention of the cannula in the sclera of the eye.

In addition, the invention relates to a fluid connector for ophthalmic surgery according to claim 5.

By providing the proximal part of the cannula receiving cavity in the connector body, an ingenious coupled structure can be obtained wherein the fluid connector is coupled to the trocar module via various coupling elements, at least including the valve unit being traversed by the capillary tube, the cannula intermediate tube portion receiving the capillary distal end, and the cavity receiving or clamping the cannula proximal part.

The connector body is provided with a multiple number of clamping elements, preferably mainly evenly distributed in a circumferential direction around the longitudinal axis of the connector body, extending inwardly into the cavity for clampingly engaging the trocar module, such as its valve unit, in the coupled state.

Generally, the fluid input or output line of the fluid connector to be connected to the proximal part of the capillary tube may e.g. be an irrigation line, an aspiration line, a viscous fluid injection line, or a viscous fluid extraction line.

Additionally, the invention relates to a trocar system for ophthalmic surgery, comprising a trocar module according to claim 1 coupled with a fluid connector according to claim 5, wherein the intermediate tube portion encloses a distal end of the capillary tube

The capillary tube of the fluid connector is then engaged by the intermediate tube portion and the valve unit of the trocar module, thus realizing an interference or frictional fit between the capillary tube on the one hand, and the intermediate tube portion and the valve unit on the other hand.

The invention also relates to a method for coupling a trocar module according to claim 1 with a fluid connector according to claim 5. Further, the invention relates to a method for decoupling a trocar system according to claim 8 into a trocar module according to claim 1 and a fluid connector according to claim 5.

The method may include a step of determining the inner diameter of the distal part of the tubular shaped cannula by performing a fluid impedance measurement at a proximal side of the capillary tube, thereby easily identifying a gauge size of the trocar module.

Further advantageous embodiments according to the invention are described in the following claims.

It should be noted that the technical features described above or below may each on its own be embodied in a trocar module, a fluid connector and/or in a method, i.e. isolated from the context in which it is described, separate from other features, or in combination with only a number of the other features described in the context in which it is disclosed. Each of these features may further be combined with any other feature disclosed, in any combination.

The invention will be further elucidated on the basis of exemplary embodiments which are represented in the drawings. The exemplary embodiments are given by way of non-limitative illustration of the invention. In the drawings:
Fig. 1 shows a schematic perspective view of a trocar module and a fluid connector according to the invention;
Fig. 2 shows a schematic cross sectional view of the trocar module and the fluid connector shown in Fig. 1, in a de-coupled state;
Fig. 3 shows a schematic cross sectional view of the trocar module and the fluid connector shown in Fig. 1, in a coupled state;
Fig. 4 shows another schematic perspective view of the trocar module shown in Fig. 1;
Fig. 5 shows a diagram with graphs of pressure drops as a function of flow in trocar modules according to the invention;
Fig. 6 shows a schematic cross sectional view of another embodiment of a fluid connector according to the invention;
Fig. 7 shows a schematic cross sectional view of an exemplary fluid connector;
Fig. 8 shows a schematic perspective view of a further embodiment of the fluid connector according to the invention;
Fig. 9 shows a schematic cross sectional view of the fluid connector shown in Fig. 8 and a trocar module, in a coupled state;
Fig. 10 shows a flow chart of a method for coupling according to the invention, and
Fig. 11 shows a flow chart of a method for coupling according to the invention.

In the figures identical or corresponding parts are represented with the same reference numerals. The drawings are only schematic representations of embodiments of the invention, which are given by manner of non-limited examples.

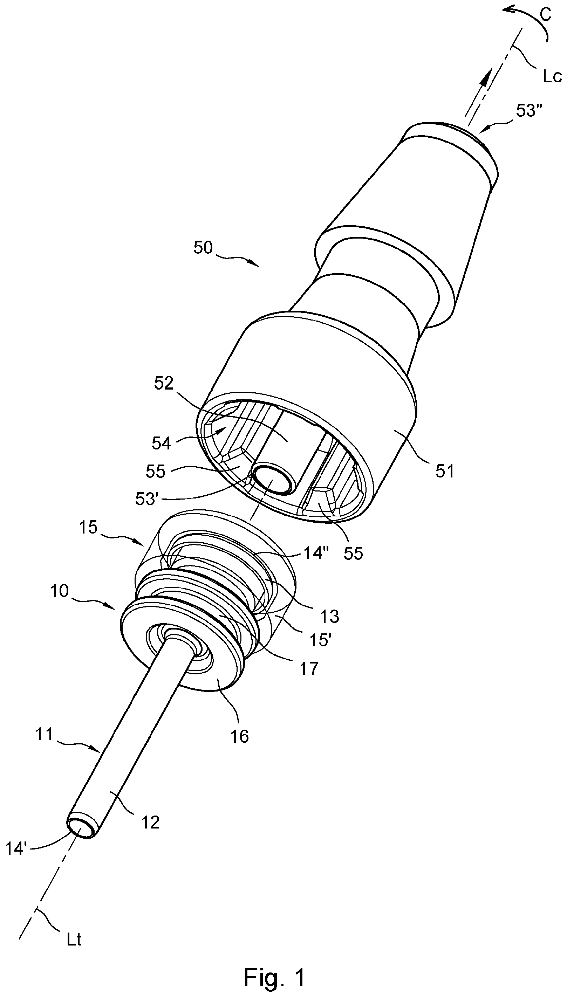

Figure 1 shows a schematic perspective view of a trocar module 10 according to the invention as well as a fluid connector 50, such as an infusion connector, according to the invention, the trocar module 10 and the fluid connector 50 being arranged for ophthalmic surgery.

The trocar module 10 has a tubular shaped cannula 11 having a distal part 12 and a proximal part 13 that are preferably together formed as an integral element. The distal part 12 has a distal end 14' and is arranged for insertion into the eye, through the eye's sclera. The proximal part 13 of the tubular shaped cannula 11 has a proximal end 14" and is arranged for receiving a capillary tube of the fluid connector 50, in a coupled state with said fluid connector 50. Typically, the cannula 11 may have a wall thickness ranging from circa 0.04 mm to circa 0.05 mm.

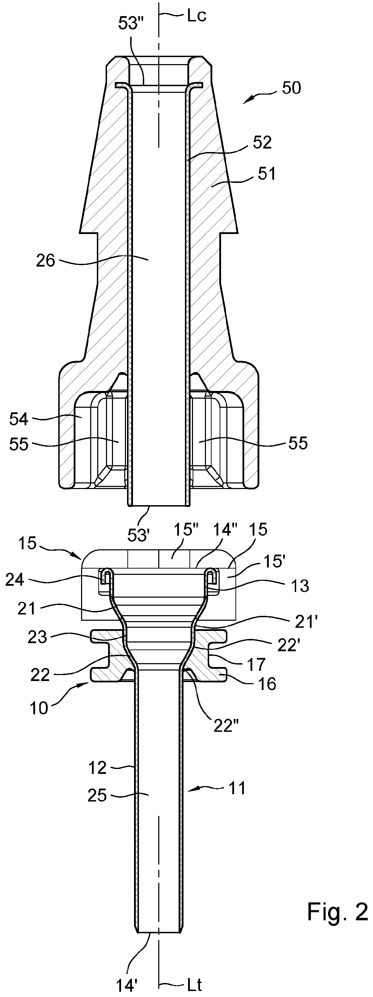

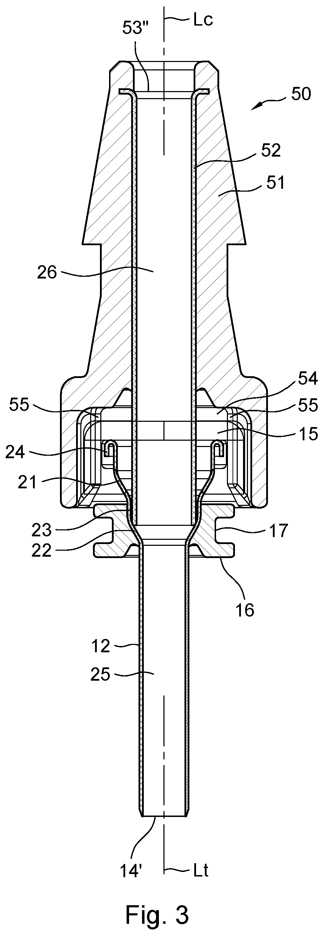

The trocar module 10 also has a valve unit 15 arranged at the proximal part 13 of the cannula 11 for, optionally, sealingly surrounding said capillary tube when received in the cannula 11, in the coupled state. In an uncoupled state, the valve unit 15 seals the cannula 11, i.e. sealingly closes off the proximal end or cannula head 14" of the tubular shaped cannula to counteract fluid leakage flowing from the interior of the eye. Further, in the shown embodiment, the valve unit 15 sealingly surrounds the cannula 11 adjacent its proximal end 14". Here, the valve unit 15 is integrally formed, having an annular shaped body 15' that is open at a first axial end and sealingly closed at a second axial end, opposite to the first axial end. The sealingly closed axial end may include a multiple number of flanges 15" that mutually overlap, are biased against each other and individually partially cover the proximal end 14", as especially visible in Fig. 2 and Fig. 3. Preferably, the valve unit 15 is removable, e.g. for enabling irrigation fluid or silicone oil to flow outwardly from the eye during specific surgery conditions. However, the valve unit 15 may be permanently mounted to the tubular shaped cannula 11. In another embodiment, the valve unit 15 does not circumscribe the cannula 11, however sealingly closing off the proximal end 14".

The valve unit 15 sealingly closes the cannula when no instrument, such as a fluid connector, is inserted into the cannula. Further, the valve unit 15 may minimize leakage when an instrument, such as a fluid connector, is inserted into the cannula.

Further, the trocar module 10 is provided with a collar 16 arranged around the cannula 11 and provided, at its exterior side with a circumferential groove 17 for engaging the sclera so as to stabilize the collar's position in the eye and for serving as an engaging structure that can be clamped by a forceps or another surgical instrument handling the trocar module 10. Alternatively, another collar 16, e.g. without circumferential groove, or another engaging structure can be applied.

In the shown embodiment, the tubular shaped cannula 11 has a mainly circular cylindrical shape being mainly rotationally symmetric with respect to its longitudinal axis Lt. Also the valve unit 15 and the collar 16 are rotationally symmetric, concentric to the longitudinal axis Lt.

The cannula 11 can e.g. be made from a metal or a metal alloy. Further, the valve unit 15 may be made from an elastic material such as a silicone material.

The fluid connector 50, also referred to as a catheter, has a generally cylindrical shaped connector body or overmoulded part 51 and a capillary tube 52, in the shown embodiment concentric to each other and relative to their common longitudinal axis Lc. The connector body 51 surrounds the capillary tube 52 extending through said connector body 51. The capillary tube 52 has a distal part 53' provided with a distal end 52', also referred to as distal tip, for coupling with the trocar module 10, and a proximal part 53" for connection with a fluid input or output line, such as an irrigation line, an aspiration line, a viscous fluid injection VFI line, or a viscous fluid extraction VFE line.

The connector body 51 is provided with a cavity 54 traversed by the capillary tube distal part 53' for receiving at least a portion of the proximal part 13 of the cannula 11, including at least a portion of the valve unit 15, in the coupled state, as explained in more detail referring to Fig. 3. Further, the connector body 51 is provided with a multiple number of clamping elements 55 extending inwardly into the cavity 54 for clampingly engaging, connecting, locking and/or fixing the trocar module 10, especially its valve unit 15, in the coupled state, as explained in more specific detail referring to Fig. 3. Preferably, the clamping elements 55 are mainly evenly distributed in a circular direction C around the capillary tube 52. The clamping elements 55 have inner surfaces facing radially inwardly, said inner surfaces being smooth or roughened to improve their clamping force by increased surface friction.

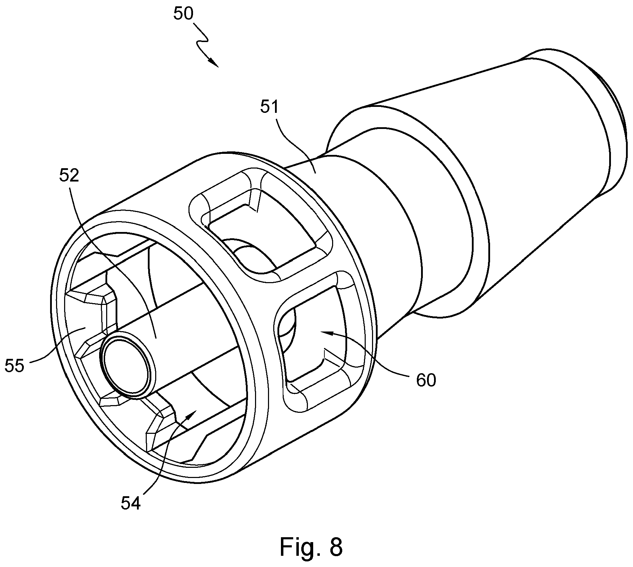

In the shown embodiment, both the connector body 51 and the cavity 54 have a mainly circular cylindrical shape being rotationally symmetric relative to their longitudinal axis Lc, the connector body 51 being formed, at its distal end, as a skirt. In the shown embodiment, the skirt has a closed circumferential contour. However, the skirt may have openings as e.g. shown in Fig. 8, e.g. between the clamping elements 55, the openings optionally extending towards and up to the distal end of the skirt, the clamping element 55 then being formed as fingers that are movable independently from each other.

In the shown embodiment, the capillary tube 52 is a separate part mounted in the connector body 51. However, in principle, the capillary tube 52 and the connector body 51 can be integrally formed, as a single unit.

Figure 2 shows a schematic cross sectional view of the trocar module 10 and the fluid connector 50 shown in Fig. 1, in a de-coupled state, while Figure 3 shows them in a coupled state, respectively. The proximal part 13 of the cannula 11 has a first tapered tube portion 21, a second tapered tube portion 22 and an intermediate tube portion 23 located between the first tapered tube portion 21 and the second tapered tube portion 22. The first tapered tube portion 21 is located at a proximal side of the intermediate tube portion 23 and is tapered towards the intermediate tube portion 23 for aligning with the capillary tube 52 during coupling. The intermediate tube portion 23 has a generally constant cross section and encloses the distal end 53' of the capillary tube 52, in the coupled state, as shown in Fig. 3. Further, the second tapered tube portion 22 tapers from the intermediate tube portion 23 towards the cannula distal part 12.

It is noted that the first tapered tube portion 21, the intermediate tube portion 23 and the second tapered portion 22 form a double tapered fluid passage or canal 25 in the cannula 11 for safely coupling the capillary tube 52 to the cannula 11 while the interior diameter of the capillary tube 52 can still be substantially the same or greater than the inner diameter of the cannula distal part 12, resulting in a flow structure wherein the capillary tube 52 does not extend or protrude into the smallest diameter structure of the cannula, its distal part 52. Then, the flow rate, generally limited by the passage having smallest internal diameter of the whole flow path, can be relatively large allowing a relatively low fluid pressure and a stable intra ocular pressure IOP, while, on the other hand, the exterior of the cannula 11 can be relatively small to minimize wound trauma.

The first tapered tube portion 21, the intermediate tube portion 23 and the second tapered portion 22 each have an inner wall defining a local contour of the cannula canal 25. Here, the complete inner wall of the intermediate tube portion contacts the distal part of the capillary tube, in the coupled state of the trocar module and the fluid connector. Then, the distal end 53' of the capillary tube abuts against the second tapered tube portion of the cannula. Alternatively, the capillary tube is not advanced until abutting the second tapered tube portion of the cannula. Then, a proximal portion of the intermediate tube portion contacts the distal part of the capillary tube. Generally, the intermediate tube portion engages at least a portion of the distal part of the capillary tube of the fluid connector.

In the shown embodiment, the first taper tube portion 21, the intermediate tube portion 23 and the second taper tube portion 22 are located directly adjacent to each other, in the indicated order. Then, a tapered end 21' of the first tapered tube portion 21 adjoins a proximal end of the intermediate tube portion 23, while a distal end of the intermediate tube portion 23 adjoins a proximal end 22' of the second tapered tube portion 22. Further, a distal end 22" of the second tapered tube portion 22 adjoins a proximal end of the cannula distal part 12. In the shown embodiment, the proximal part 13 of the cannula 11 includes a double tapered tube portions 21 that are mutually connected via the intermediate tube portion 23. In principle, another structure may be applied between said tapered and intermediate tube portions 21, 22, 23, e.g. an annular clamping interface tube portion between the first tapered tube portion 21 and the intermediate tube portion 23.

Further, in the shown embodiment, the proximal part 13 of the cannula 11 has an extended tube portion 24 between the proximal end 14" of the cannula 11 and the first tapered tube portion 21, the extended tube portion 24 having a generally constant cross section.

During a process of coupling the trocar module 10 with the fluid connector 50, the capillary tube 52 of the fluid connector 50 is received in the proximal part 13 of the tubular shaped cannula 11 of the trocar module 10, thereby receiving at least a portion of the proximal part 13 of the cannula 11, including its valve unit 15, in the cavity 54 of the connector body 51 of the fluid connector 50. Further, the capillary tube 52 of the fluid connector 50 is advanced into the cannula 11 until the intermediate tube portion 23 encloses a distal tube portion 52' of the capillary tube 52.

In the coupled state, shown in Fig. 3, the trocar module 10 and the fluid connector 50 form a trocar system. Here, the intermediate tube portion 23 encloses the distal end or distal tip 52' of the capillary tube 52. Further, the capillary tube 52 of the fluid connector 50 is engaged by both said intermediate tube portion 23 and the valve unit 15 of the trocar module 10. Also, the skirt of the connector body, especially the clamping elements 55 thereon, clamp the proximal part 13 of the cannula 11, including its valve unit 15. Preferably, the radially facing inner surfaces of the clamping elements 55 define a circle having a smaller diameter than the outer diameter of the valve unit 15, such that, in the coupled state, the clamping elements 55 are pressed into the preferably elastic material of the valve unit 15.

Preferably, an inner diameter of the capillary tube 52, enclosing a capillary fluid passage or canal 26, is substantially the same or greater than an inner diameter of the cannula distal part 12 so that the fluid impedance of the capillary tube 52 does not significantly contribute to the overall fluid impedance, thereby exploiting the fluid flow performance of the fluid passage or canal 25 in the cannula 11 in an optimal manner.

Similarly, an inner diameter of the intermediate tube portion 23 is substantially the same or greater than an outer diameter of the cannula distal part 12, thereby reducing the fluid impedance of the capillary tube 52.

During a process of decoupling or uncoupling a trocar system back into a trocar module 10 and a fluid connector 50 as separate components, the capillary tube 52 of the fluid connector 50 is retracted from the cannula 11 of the trocar module 10 until the capillary tube 52 is released by the intermediate tube portion 23 and the valve unit 15, and the proximal part 13 of the cannula 11, including its valve unit 15, is released by the skirt of the connector body 51.

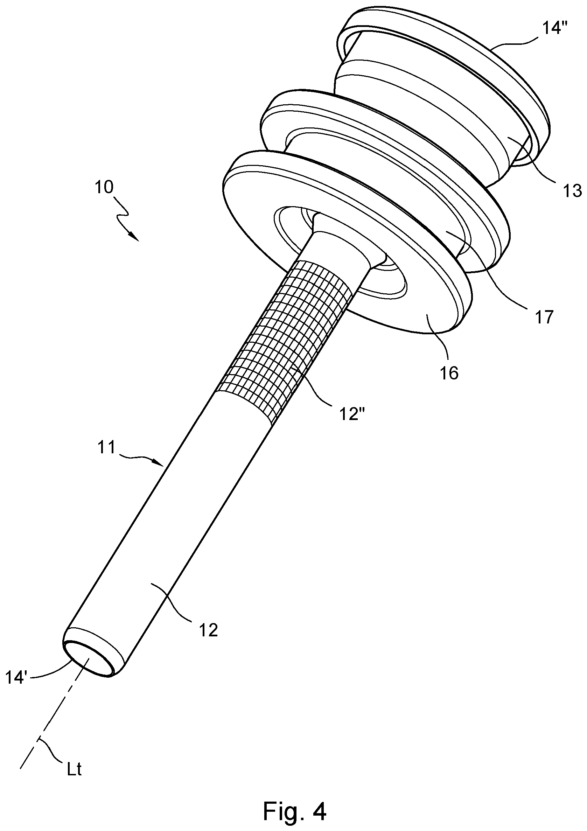

Figure 4 shows another schematic perspective view of the trocar module 10 shown in Fig. 1. Here, the valve unit 15 has been removed. As shown, the distal part 12 of the tubular shaped cannula 11 has an exterior surface 12' that is at least partly provided with a laser fabricated texture structure 12" or pattern. By providing laser texture structures 12" an exterior surface 12' having increased surface roughness or surface pattern is obtained improving retention of the cannula 11 in the sclera of the eye. On the other hand, by applying a laser process, it is counteracted, contrary to rolling and/or machining processes, that damage, such as excessive deformation at the edges of the surface or impact on circularity risk to tool insertion, occurs to the cannula that is typically thin walled, especially if the cannula 11 has a relatively small gauge size. Preferably, the surface has a graded roughness so as to avoid that wound damage or a non-smooth insertion force during surgery is counteracted. Further, the laser fabricated texture structure or pattern may have a regular structure such as a repeated roughness profile. According to an aspect a process of preparing a trocar module for ophthalmic surgery is provided, the trocar module comprising a tubular shaped cannula having a distal part for insertion through the eye's sclera and a proximal part for receiving a capillary tube of a fluid connector in a coupled state with said fluid connector, the process comprising exposing an exterior surface of the tubular shaped cannula to laser radiation for generating a texture structure on at least a part of the exterior surface. Advantageously, the laser process is applied for optimizing or finely tuning the texture structure.

It is noted however that, in principle, the exterior surface 12' of the distal part 12 of the tubular shaped cannula 11 may have another texture pattern, e.g. provided by a rolling and/or machining process, or no texture pattern at all.

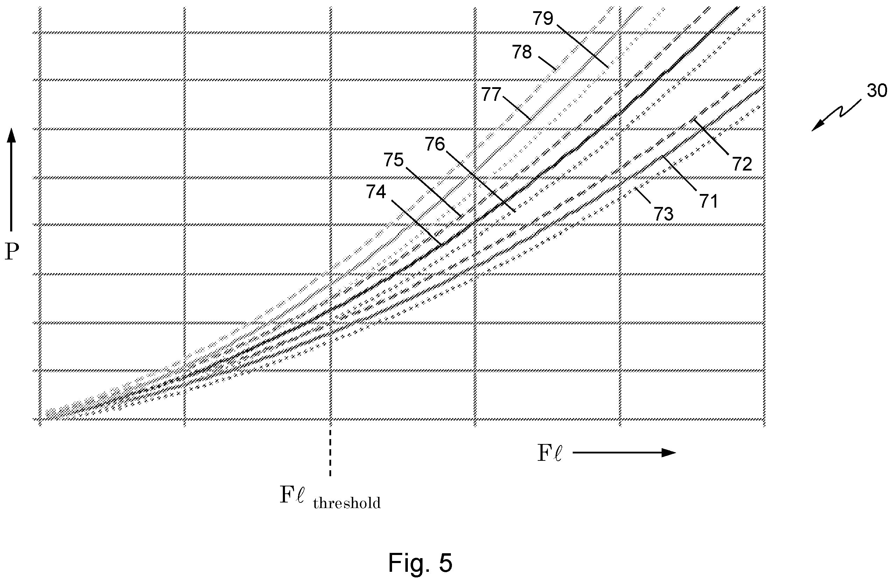

Figure 5 shows a diagram 30 with graphs of pressure drops as a function of flow in trocar modules 10 according to the invention. Specifically, a pressure drop P in mmHg is depicted as a function of flow Fl in ml/min. A first pressure drop graph 71 corresponds to a trocar unit having a first internal diameter or gauge size D1. Typically, effective internal diameters of trocar units falling in the same gauge class may deviate due to manufacturing tolerances. Then, also corresponding pressure drops may deviate. The first pressure drop graph 71 reflects an average pressure drop behaviour of a group trocar units having the first internal diameter or gauge size D1, also referred to as a first set of trocar units. A second pressure drop graph 72 corresponds to another trocar module 10 of the first set of trocar units, however having a maximum pressure drop behaviour. Similarly, a third pressure drop graph 73 corresponds to yet another trocar module of the first set of trocar units, however having a minimum pressure drop behaviour. Then, the first set of trocar units having the first internal diameter or gauge size have a pressure drop curve that statistically deviates in a bandwidth between the second and the third pressure drop graphs 72, 73, in a statistical sense, e.g. defined by a percentage, e.g. 95%, of its total population distribution.

Similarly, a second set of trocar units having a second internal diameter or gauge size D2, larger than the first internal diameter or gauge size D 1, have a pressure drop that on average corresponds to a fourth pressure drop graph 74 shown in the diagram 30, and statistically may deviate between a fifth pressure drop graph 75, corresponding to a maximum pressure drop, and a sixth pressure drop graph 76, corresponding to a minimum pressure drop.

Also, a third set of trocar units having a second internal diameter or gauge size D3, larger than the second internal diameter or gauge size D2, have a pressure drop that on average corresponds to a seventh pressure drop graph 77 shown in the diagram 30, and statistically may deviate between a eight pressure drop graph 78, corresponding to a maximum pressure drop, and a ninth pressure drop graph 79, corresponding to a minimum pressure drop.

As shown in the diagram 30 of Fig. 5, pressure drop graphs of the first, second and third set of trocar units do not overlap above a threshold flow level Fl_{threshold}. Therefore, there may be a need to easily identify the first, second and third set of trocar units, in a surgical context, as the respective sets of trocar units would be used with corresponding sets of other ophthalmic surgical devices so as to counteract a sub-optimal treatment of the eye under surgery. By identifying the gauge size of the trocar units, preferably before or at the start of surgery, the surgical system setting scan be set such as to optimize the intraocular pressure IOP and IOP stability during surgery. As an example, the first set of trocar units would be used in combination with a phaco vitrectomy device having a relatively small internal diameter or relatively large gauge size, preferably not in combination with a phaco vitrectomy device having a relatively large internal diameter or relatively small gauge size, so as to avoid that an infusion or aspiration process takes too much time. As a further example, the third set of trocar units would be used in combination with an ophthalmic surgery device having a relatively large internal diameter or relatively small gauge size, preferably not in combination with a phaco vitrectomy device having a relatively small internal diameter or relatively large gauge size, so as to avoid that an infusion or aspiration process occurs with a fluid flow that is too large possibly causing damage to the eye.

For the purpose of identify a trocar module, the inner diameter of the distal part 12 of the tubular shaped cannula 11, or its gauge size, can be determined by performing a fluid impedance measurement at a proximal side of the capillary tube 52 of the fluid connector, preferably after coupling with the fluid connector 50. By evaluating a measured fluid impedance or pressure drop P per flow FL, a corresponding effective inner diameter or gauge size can be found, thereby identifying the trocar module.

It is noted that the step of identifying a trocar module can not only be performed on a trocar module according to claim 1, but also more generally on a trocar module for ophthalmic surgery, comprising a tubular shaped cannula having a distal part for insertion through the eye's sclera and a proximal part for receiving a capillary tube of a fluid connector in a coupled state with said fluid connector.

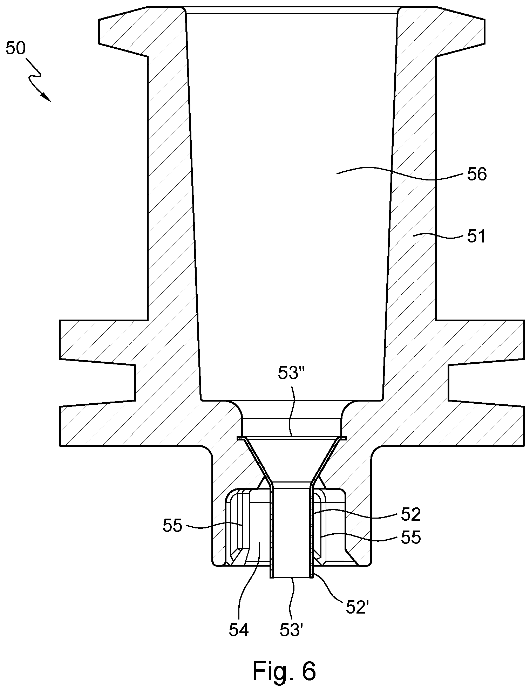

Figure 6 shows a schematic cross sectional view of another embodiment of a fluid connector 50 according to the invention. Similar to the fluid connector described referring to Fig. 1, the fluid connector includes a connector body 51 and a capillary tube 52 surrounded by said connector body 51. The connector body 51 is provided with a cavity 54 for receiving at least a portion of the proximal part 13 of the cannula 11, in the coupled state. The connector body 51 also has a multiple number of clamping elements 55 extending inwardly into the cavity 54 for clampingly engaging the trocar module 10, in the coupled state.

The capillary tube 52 has a distal part 53' traversing the cavity 54 and being provided with a distal end or distal tip 52' for coupling with a trocar module 10, and a proximal part 53". Here, the proximal part 53" is arranged for connection with a viscous fluid extraction VFE line. The connector body 51 is further provided with a proximal cavity 56 for housing a distal end the VFE line connected to the capillary 52.

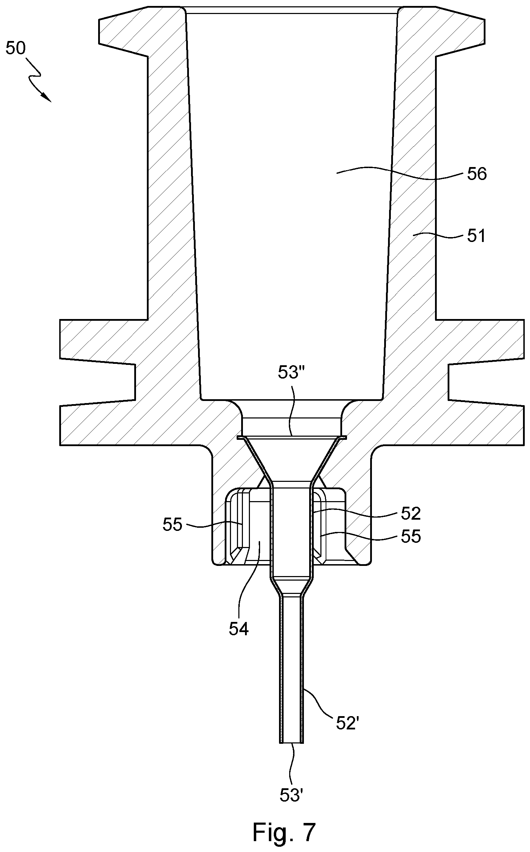

Figure 7 shows a schematic cross sectional view of an exemplary fluid connector 50. The fluid connector 50 has a design similar to the fluid connector 50 shown in Fig. 6, however, the proximal part 53" is now arranged for connection with a viscous fluid injection VFI line. Further, the distal tip 52' of the capillary 52 is significantly longer than in the embodiment shown in Fig. 6 allowing the distal tip 52' to traverse, through the proximal part 13 of the tubular shaped cannula 11, a substantial portion of the distal part 12 of the tubular shaped cannula 11, thereby allowing a greater internal over pressure without the fluid connector 50 and the coupled trocar module 10 being decoupled and removed from each other.

Figure 8 shows a schematic perspective view of a further embodiment of the fluid connector 50 according to the invention. Similar to the fluid connector described referring to Fig. 6 and 7, the fluid connector 50 includes a connector body 51 and a capillary tube 52 surrounded by said connector body 51. The connector body 51 is provided with a cavity 54 for receiving at least a portion of the proximal part 13 of the cannula 11, in the coupled state. The connector body 51 also has a multiple number of clamping elements 55 extending inwardly into the cavity 54 for clampingly engaging the trocar module 10, in the coupled state.

The connector body 51 is formed, at its distal end, as a skirt. In the shown embodiment, the skirt has openings 60 to receive circumferential portions of the valve unit 15 of the trocar module 10, in the coupled state. Here, the valve unit 15 is allowed to extend slightly into said openings 60. Further, the clamping elements 55 form an undercut engaging below the valve unit 15, thereby releasably locking the trocar module 10 to the fluid connector 50, in the coupled state.

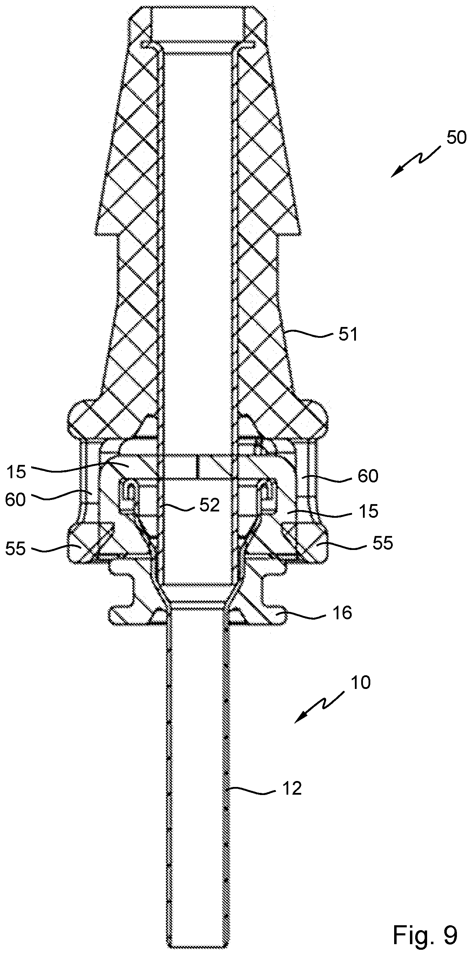

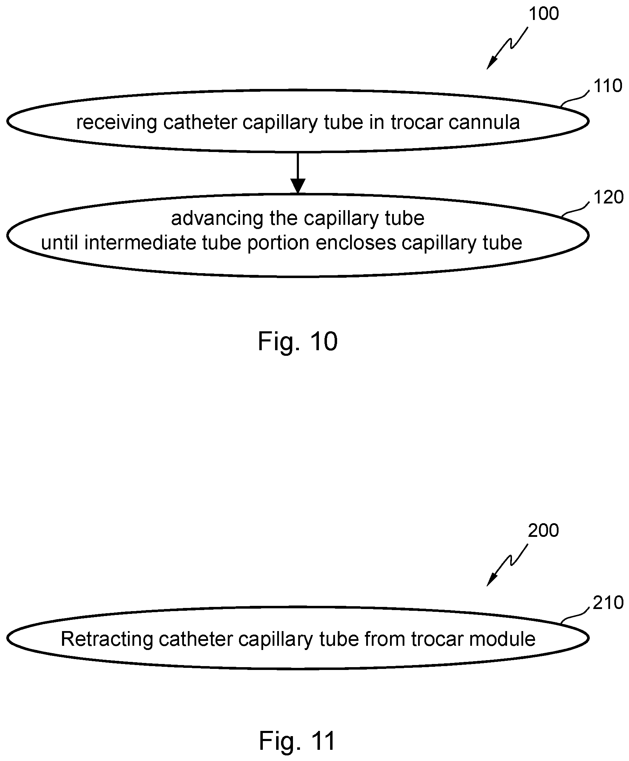

Figure 9 shows a schematic cross sectional view of the fluid connector 50 shown in Fig. 8 and a trocar module 10, in the coupled state. As clearly visible in Fig. 9, the fingers 55 engage around and below the valve unit 15 of the trocar module 10.Figure 10 shows a flow chart of a method according to the invention. The method is used for coupling a trocar module with a fluid connector so as to form a trocar system. The method 100 comprises a step of receiving 110 the capillary tube of the fluid connector in the proximal part of the tubular shaped cannula of the trocar module, thereby receiving at least a portion of the proximal part of the cannula in the cavity of the connector body of the fluid connector, and a step of advancing 120 the capillary tube of the fluid connector into the cannula until the intermediate tube portion encloses a distal end of the capillary tube. Preferably, the method also comprises a step of advancing the capillary tube of the fluid connector into the cannula until the distal end of the capillary tube abuts against the second tapered tube portion of the cannula.

The method 100 may further comprise a step of determining the inner diameter of the distal part 12 of the tubular shaped cannula 11 by performing a fluid impedance measurement at a proximal side of the capillary tube 52 of the fluid connector, e.g. for identifying a gauge size of the trocar module 10 as described above.

Figure 11 shows a flow chart of a method 200 according to the invention. The method is used for decoupling a trocar system into a trocar module 10 and a separate fluid connector 50. The method 200 comprises a step of retracting 210 the capillary tube 52 of the fluid connector 50 from the cannula 11 of the trocar module 10.

The invention is not restricted to the embodiments described herein. It will be understood that many variants are possible.

It is noted that the trocar module described above can not only be used for coupling to a corresponding fluid connector, but also for being traversed by other ophthalmic surgical devices such as a laser device, a vitrectome or a forceps.

These and other embodiments will be apparent for the person skilled in the art and are considered to fall within the scope of the invention as defined in the following claims. For the purpose of clarity and a concise description features are described herein as part of the same or separate embodiments. However, it will be appreciated that the scope of the invention may include embodiments having combinations of all or some of the features described.

## Claims

1. A trocar module (10) for ophthalmic surgery, comprising:
- a tubular shaped cannula (11) having a distal part (12) for insertion through the eye's sclera and a proximal part (13) for receiving a capillary tube (52) of a fluid connector (50) in a coupled state with said fluid connector (50), and
- a valve unit (15) arranged at the proximal part (13) of the cannula (11) for sealing said cannula (11) in an uncoupled state,
wherein the proximal part (13) of the cannula (11) has a first tapered tube portion (21), a second tapered tube portion (22) and an intermediate tube portion (23) located between the first tapered tube portion (21) and the second tapered tube portion (22), wherein the first tapered tube portion (21) is located at a proximal side of the intermediate tube portion (23) and is tapered towards the intermediate tube portion (23) for aligning with the capillary tube (52) during coupling,
wherein the intermediate tube portion (23) has a generally constant cross section and encloses and engages at least a portion of a distal end (53') of the capillary tube (52) in the coupled state, and wherein the second tapered tube portion (22) tapers from the intermediate tube portion (23) towards the cannula distal part (12), wherein the capillary tube distal end (53'), in the coupled state, abuts against the second tapered tube portion (22),
wherein the first tapered tube portion (21), the intermediate tube portion (23) and the second tapered tube portion (22) form a double tapered canal (25) in the cannula (11),
wherein the valve unit sealingly surrounds the cannula adjacent a proximal end of the tubular shaped cannula,
wherein, in the coupled state, the capillary tube of the fluid connector is engaged by the intermediate tube portion and the valve unit of the trocar module.

2. A trocar module according to claim 1, wherein an inner diameter of the capillary tube is substantially the same or greater than an inner diameter of the cannula distal part.

3. A trocar module according to any of the preceding claims, wherein an inner diameter of the intermediate tube portion is substantially the same or greater than an outer diameter of the cannula distal part.

4. A trocar module according to any of the preceding claims, further comprising a collar arranged around the cannula and provided with, at its exterior side with a circumferential groove.

5. A fluid connector (50) for ophthalmic surgery, comprising a capillary tube (52) and a connector body (51) surrounding said capillary tube (52), the capillary tube (52) having a proximal part (53") for connection with a fluid input or output line, and a distal part (53') for coupling with a trocar module (10) according to claim 1, wherein the connector body (51) is provided with a cavity (54) traversed by the capillary tube distal part (53') for receiving at least a portion of the proximal part (13) of the cannula (11), in the coupled state, wherein the valve unit (15) of the trocar module (10) is made from an elastic material, and wherein the connector body (51) is provided with a multiple number of clamping elements (55) extending inwardly into the cavity (54) for clampingly engaging the valve unit (15), in the coupled state.

6. A fluid connector according to claim 5, wherein the clamping elements are mainly evenly distributed in a circular direction around the capillary tube.

7. A fluid connector according to any of the preceding claims 5 or 6, wherein the fluid input or output line to be connected to the proximal part of the capillary tube is an irrigation line, an aspiration line, a viscous fluid injection line, or a viscous fluid extraction line.

8. A trocar system for ophthalmic surgery, comprising a trocar module according to claim 1 and a fluid connector according to claim 5, wherein, in the coupled state, the capillary tube distal end (53') against which the second tapered tube portion (22) abuts is the capillary tube distal end (53') of said fluid connector.

9. A trocar system according to any of the preceding claims 8 , wherein the intermediate tube portion has an inner wall defining a local contour of the cannula canal and wherein the complete inner wall of the intermediate tube portion contacts the distal part of the capillary tube.

10. A method for coupling a trocar module (10) according to claim 1 with a fluid connector (50) according to claim 5 so as to form a trocar system according to claim 8, the method comprising the steps of:
- receiving the capillary tube (52) of the fluid connector (50) in the proximal part (13) of the tubular shaped cannula (11) of the trocar module (10), thereby receiving at least a portion of the proximal part (13) of the cannula (11) in the cavity (54) of the connector body (51) of the fluid connector (50), and
- advancing the capillary tube (52) of the fluid connector (50) into the cannula (11) until the intermediate tube portion (23) encloses and engages at least a portion of a distal tip (52') of the capillary tube (52),
further comprising the step of:
- advancing the capillary tube of the fluid connector into the cannula until the distal end of the capillary tube abuts against the second tapered tube portion of the cannula.

11. A method according to claim 10, further comprising the step of determining the inner diameter of the distal part of the tubular shaped cannula by performing a fluid impedance measurement at a proximal side of the capillary tube.

12. A method for decoupling a trocar system according to claim 8 from its coupled state into a trocar module according to claim 1 and a fluid connector according to claim 5, comprising the step of retracting the capillary tube of the fluid connector from the cannula of the trocar module.

## Patentansprüche

1. Trokarmodul (10) für ophthalmische Chirurgie, umfassend:
- eine röhrenförmige Kanüle (11) mit einem distalen Teil (12) zum Einführen durch die Sklera des Auges und einem proximalen Teil (13) zum Aufnehmen einer Kapillarröhre (52) eines Fluidverbinders (50) in einem gekoppelten Zustand mit dem Fluidverbinder (50) und
- eine Ventileinheit (15), die an dem proximalen Teil (13) der Kanüle (11) angeordnet ist, um die Kanüle (11) in einem entkoppelten Zustand abzudichten,
wobei der proximale Teil (13) der Kanüle (11) einen ersten sich verjüngenden Rohrabschnitt (21), einen zweiten sich verjüngenden Rohrabschnitt (22) und einen Zwischenrohrabschnitt (23) aufweist, der sich zwischen dem ersten sich verjüngenden Rohrabschnitt (21) und dem zweiten sich verjüngenden Rohrabschnitt (22) befindet, wobei sich der erste sich verjüngende Rohrabschnitt (21) an einer proximalen Seite des Zwischenrohrabschnitts (23) befindet und sich zum Ausrichten auf die Kapillarröhre (52) während des Koppelns zu dem Zwischenrohrabschnitt (23) hin verjüngt,
wobei der Zwischenrohrabschnitt (23) einen im Allgemeinen konstanten Querschnitt aufweist und mindestens einen Abschnitt eines distalen Endes (53') der Kapillarröhre (52) in dem gekoppelten Zustand umschließt und diesen in Eingriff nimmt und wobei sich der zweite sich verjüngende Rohrabschnitt (22) von dem Zwischenrohrabschnitt (23) zu dem distalen Kanülenteil (12) hin verjüngt, wobei das distale Kapillarröhrenende (53') im gekoppelten Zustand an dem zweiten sich verjüngenden Rohrabschnitt (22) anliegt, wobei der erste sich verjüngende Rohrabschnitt (21), der Zwischenrohrabschnitt (23) und der zweite sich verjüngende Rohrabschnitt (22) einen doppelten sich verjüngenden Kanal (25) in der Kanüle (11) bilden,
wobei die Ventileinheit die Kanüle einem proximalen Ende der röhrenförmigen Kanüle benachbart abdichtend umgibt,
wobei die Kapillarröhre des Fluidverbinders in dem gekoppelten Zustand von dem Zwischenrohrabschnitt und der Ventileinheit des Trokarmoduls in Eingriff genommen ist.

2. Trokarmodul nach Anspruch 1, wobei ein Innendurchmesser der Kapillarröhre im Wesentlichen gleich einem oder größer als ein Innendurchmesser des distalen Kanülenteils ist.

3. Trokarmodul nach einem der vorhergehenden Ansprüche, wobei ein Innendurchmesser des Zwischenrohrabschnitts im Wesentlichen gleich einem oder größer als ein Außendurchmesser des distalen Kanülenteils ist.

4. Trokarmodul nach einem der vorhergehenden Ansprüche, ferner umfassend einen Bund, der um die Kanüle herum angeordnet ist und an seiner Außenseite mit einer Umfangsnut versehen ist.

5. Fluidverbinder (50) für ophthalmische Chirurgie, umfassend eine Kapillarröhre (52) und einen Verbinderkörper (51), der die Kapillarröhre (52) umgibt, wobei die Kapillarröhre (52) einen proximalen Teil (53") zum Verbinden mit einer Fluideingangs- oder -ausgangsleitung und einen distalen Teil (53') zum Koppeln mit einem Trokarmodul (10) nach Anspruch 1 aufweist, wobei der Verbinderkörper (51) mit einem Hohlraum (54) versehen ist, der von dem distalen Teil (53') der Kapillarröhre durchquert wird, um mindestens einen Abschnitt des proximalen Teils (13) der Kanüle (11) im gekoppelten Zustand aufzunehmen, wobei die Ventileinheit (15) des Trokarmoduls (10) aus einem elastischen Material hergestellt ist und wobei der Verbinderkörper (51) mit einer Vielzahl von Klemmelementen (55) versehen ist, die sich im gekoppelten Zustand nach innen in den Hohlraum (54) erstrecken, um die Ventileinheit (15) in Klemmeingriff zu bringen.

6. Fluidverbinder nach Anspruch 5, wobei die Klemmelemente hauptsächlich gleichmäßig in einer kreisförmigen Richtung um die Kapillarröhre verteilt sind.

7. Fluidverbinder nach einem der vorhergehenden Ansprüche 5 oder 6, wobei die Fluideingangs- oder -ausgangsleitung, die mit dem proximalen Teil der Kapillarröhre verbunden werden soll, eine Irrigationsleitung, eine Aspirationsleitung, eine Leitung zum Einspritzen von viskosem Fluid oder eine Leitung zum Abziehen von viskosem Fluid ist.

8. Trokarsystem für ophthalmische Chirurgie, umfassend ein Trokarmodul nach Anspruch 1 und einen Fluidverbinder nach Anspruch 5, wobei im gekoppelten Zustand das distale Ende (53') der Kapillarröhre, an dem der zweite sich verjüngende Rohrabschnitt (22) anliegt, das distale Kapillarröhrenende (53') des Fluidverbinders ist.

9. Trokarsystem nach einem der vorhergehenden Ansprüche 8, wobei der Zwischenrohrabschnitt eine Innenwand aufweist, die eine lokale Kontur des Kanülenkanals definiert, und wobei die vollständige Innenwand des Zwischenrohrabschnitts den distalen Teil der Kapillarröhre kontaktiert.

10. Verfahren zum Koppeln eines Trokarmoduls (10) nach Anspruch 1 mit einem Fluidverbinder (50) nach Anspruch 5, um ein Trokarsystem nach Anspruch 8 zu bilden, wobei das Verfahren die folgenden Schritte umfasst:
- Aufnehmen der Kapillarröhre (52) des Fluidverbinders (50) in dem proximalen Teil (13) der röhrenförmigen Kanüle (11) des Trokarmoduls (10), wodurch mindestens ein Teil des proximalen Teils (13) der Kanüle (11) in dem Hohlraum (54) des Verbinderkörpers (51) des Fluidverbinders (50) aufgenommen wird, und
- Vorschieben der Kapillarröhre (52) des Fluidverbinders (50) in die Kanüle (11), bis der Zwischenrohrabschnitt (23) mindestens einen Abschnitt einer distalen Spitze (52') der Kapillarröhre (52) umschließt und in Eingriff nimmt, ferner umfassend den folgenden Schritt:
- Vorschieben der Kapillarröhre des Fluidverbinders in die Kanüle, bis das distale Ende der Kapillarröhre an dem zweiten sich verjüngenden Rohrabschnitt der Kanüle anliegt.

11. Verfahren nach Anspruch 10, ferner umfassend den Schritt des Bestimmens des Innendurchmessers des distalen Teils der röhrenförmigen Kanüle durch Durchführen einer Flüssigkeitsimpedanzmessung an einer proximalen Seite der Kapillarröhre.

12. Verfahren zum Entkoppeln eines Trokarsystems nach Anspruch 8 aus seinem in ein Trokarmodul nach Anspruch 1 und einen Fluidverbinder nach Anspruch 5 gekoppelten Zustand, umfassend den Schritt des Zurückziehens der Kapillarröhre des Fluidverbinders aus der Kanüle des Trokarmoduls.

## Revendications

1. Module de trocart (10) pour chirurgie ophtalmique, comprenant :
- une canule de forme tubulaire (11) ayant une partie distale (12) pour l'insertion à travers la sclère de l'œil et une partie proximale (13) pour recevoir un tube capillaire (52) d'un raccord fluidique (50) dans un état couplé audit raccord fluidique (50), et
- une unité valve (15) agencée au niveau de la partie proximale (13) de la canule (11) pour effectuer l'étanchéité de ladite canule (11) dans un état découplé, dans lequel la partie proximale (13) de la canule (11) a une première portion de tube tronconique (21), une seconde portion de tube tronconique (22) et une portion de tube intermédiaire (23) située entre la première portion de tube tronconique (21) et la seconde portion de tube tronconique (22), dans lequel la première portion de tube tronconique (21) est située au niveau d'un côté proximal de la portion de tube intermédiaire (23) et présente une tronconicité vers la portion de tube intermédiaire (23) pour s'aligner avec le tube capillaire (52) durant le couplage,
dans lequel la portion de tube intermédiaire (23) a une section transversale généralement constante et encercle au moins une portion, et entre en prise avec celle-ci, d'une extrémité distale (53') du tube capillaire (52) dans l'état couplé, et dans lequel la seconde portion de tube tronconique (22) présente une tronconicité depuis la portion de tube intermédiaire (23) vers la partie distale de canule (12), dans lequel l'extrémité distale de tube capillaire (53'), dans l'état couplé, vient en butée contre la seconde portion de tube tronconique (22), dans lequel la première portion de tube tronconique (21), la portion de tube intermédiaire (23), et la seconde portion de tube tronconique (22) forment un canal tronconique double (25) dans la canule (11),
dans lequel l'unité valve entoure la canule de manière étanche, de façon adjacente à une extrémité proximale de la canule de forme tubulaire,
dans lequel, dans l'état couplé, la portion de tube intermédiaire et l'unité valve du module de trocart entrent en prise avec le tube capillaire du raccord fluidique.

2. Module de trocart selon la revendication 1, dans lequel un diamètre intérieur du tube capillaire est sensiblement identique ou supérieur à un diamètre intérieur de la partie distale de canule.

3. Module de trocart selon de quelconques des revendications précédentes, dans lequel un diamètre intérieur de la portion de tube intermédiaire est sensiblement identique ou supérieur à un diamètre extérieur de la partie distale de canule.

4. Module de trocart selon de quelconques des revendications précédentes, comprenant en outre un collier agencé autour de la canule et pourvu, au niveau de son côté extérieur, d'une rainure circonférentielle.

5. Raccord fluidique (50) pour chirurgie ophtalmique, comprenant un tube capillaire (52) et un corps de raccord (51) entourant ledit tube capillaire (52), le tube capillaire (52) ayant une partie proximale (53") pour le raccordement à une conduite d'entrée ou de sortie fluidique, et une partie distale (53') pour le couplage à un module de trocart (10) selon la revendication 1, dans lequel le corps de raccord (51) est pourvu d'une cavité (54) traversée par le tube capillaire partie distale (53') pour recevoir au moins une portion de la partie proximale (13) de la canule (11), dans l'état couplé, dans lequel l'unité valve (15) du module de trocart (10) est faite d'un matériau élastique, et dans lequel le corps de raccord (51) est pourvu d'un nombre multiple d'éléments de serrage (55) s'étendant vers l'intérieur jusque dans la cavité (54) pour entrer en prise de manière serrante avec l'unité valve (15), dans l'état couplé.

6. Raccord fluidique selon la revendication 5, dans lequel les éléments de serrage sont distribués principalement uniformément dans une direction circulaire autour du tube capillaire.

7. Raccord fluidique selon de quelconques des revendications précédentes 5 ou 6, dans lequel la conduite d'entrée ou de sortie fluidique destinée à être raccordée à la partie proximale du tube capillaire est une conduite d'irrigation, une conduite d'aspiration, une conduite d'injection de fluide visqueux, ou une conduite d'extraction de fluide visqueux.

8. Système de trocart pour chirurgie ophtalmique, comprenant un module de trocart selon la revendication 1 et un raccord fluidique selon la revendication 5, dans lequel, dans l'état couplé, l'extrémité distale de tube capillaire (53') contre laquelle vient en butée la seconde portion de tube tronconique (22) est l'extrémité distale de tube capillaire (53') dudit raccord fluidique.

9. Système de trocart selon de quelconques des revendications précédentes 8, dans lequel la portion de tube intermédiaire a une paroi intérieure définissant un contour local de la canule canal et dans lequel la paroi intérieure complète de la portion de tube intermédiaire entre en contact avec la partie distale du tube capillaire.

10. Procédé pour coupler un module de trocart (10) selon la revendication 1 à un raccord fluidique (50) selon la revendication 5 afin de former un système de trocart selon la revendication 8, le procédé comprenant les étapes suivantes :
- la réception du tube capillaire (52) du raccord fluidique (50) dans la partie proximale (13) de la canule de forme tubulaire (11) du module de trocart (10), ainsi recevant au moins une portion de la partie proximale (13) de la canule (11) dans la cavité (54) du corps de raccord (51) du raccord fluidique (50), et
- l'avance du tube capillaire (52) du raccord fluidique (50) jusque dans la canule (11) jusqu'à ce que la portion de tube intermédiaire (23) encercle au moins une portion, et entre en prise avec celle-ci, d'un bout distal (52') du tube capillaire (52), comprenant en outre l'étape suivante :
- l'avance du tube capillaire du raccord fluidique jusque dans la canule jusqu'à ce que l'extrémité distale du tube capillaire vienne en butée contre la seconde portion de tube tronconique de la canule.

11. Procédé selon la revendication 10, comprenant en outre l'étape de la détermination du diamètre intérieur de la partie distale de la canule de forme tubulaire en réalisant une mesure d'impédance fluide au niveau d'un côté proximal du tube capillaire.

12. Procédé pour découpler un système de trocart selon la revendication 8 de son état couplé dans un module de trocart selon la revendication 1 et un raccord fluidique selon la revendication 5, comprenant l'étape du retrait, depuis la canule du module de trocart, du tube capillaire du raccord fluidique.
